# EUROPÄISCHE PATENTANMELDUNG

(11) **EP 0 638 619 A2**
(43) Veröffentlichungstag der Anmeldung: **15.02.1995**
(21) Anmeldenummer: 94112338.2
(22) Anmeldetag: 08.08.1994
(51) Int. Cl.: C09D 11/00

(54) **Magnetisches Tintenkonzentrat**

(30) Priorität: 13.08.1993 DE 4327223
(71) Anmelder: BASF Aktiengesellschaft, D-67063 Ludwigshafen (DE)
(72) Erfinder: Kormann, Claudius, Dr., D-67105 Schifferstadt (DE); Schwab, Ekkehard, Dr., D-67434 Neustadt (DE); Funhoff, Angelika, Dr., D-69126 Heidelberg (DE); Boeckh, Dieter, Dr., D-67117 Limburgerhof (DE); Kroner, Matthias, Dr., D-67304 Eisenberg (DE)

(57) **Zusammenfassung**

Beschrieben sind magnetische Tintenkonzentrate, bestehend aus einer Dispersion superparamagnetischer oder hartmagnetischer Feststoffteilchen, welche in Wasser oder in Alkohol mit einer DK von ≧ 20 in Gegenwart dispergierender Substanzen vorliegen und wobei die dispergierenden Substanzen Polyelektrolyte sind, welche ein Molekulargewicht zwischen 500 und 100000 besitzen, die in der Hauptkette mindestens zwei gleiche oder verschiedene Heteroatome und in der Seitenkette mindestens drei freie Carbonsäuregruppen oder drei freie Aminogruppen enthalten. Derartige Polyelektrolyte sind leicht biologisch abbaubar. Diese Tintenkonzentrate haben günstige Eigenschaften, vor allen Dingen bezüglich Sedimentationsstabilität.

## Beschreibung

Die Erfindung betrifft ein magnetisches Tintenkonzentrat, im wesentlichen bestehend aus einer Dispersion magnetischer Feststoffteilchen in Wasser oder Alkohol in Gegenwart dispergierender Substanzen, wobei diese mindestens einen Polyelektrolyten enthalten.

Ein magnetisches Tintenkonzentrat der oben genannten gattungsmäßigen Art ist bereits aus der DE-OS 41 15 608 bekannt. Dabei werden superparamagnetische Feststoffteilchen in Wasser oder Alkohol und dispergierend wirkenden Polyelektrolyten mit einem Molekulargewicht zwischen 1000 und 25000 beschrieben. Der Polyelektrolyt ist ausgewählt aus der Gruppe aus Polyacrylat, Acrylsäure-Acrylamid-Copolymeren und Polyvinylphosphonsäure sowie der Alkalisalze dieser Verbindungen. Es handelt sich bei diesen Polyelektrolyten um Stoffe, deren biologische Abbaubarkeit nur minimal ist, so daß die Entsorgung der magnetischen Tintenkonzentrate unter heutigen ökologischen Gesichtspunkten nicht mehr optimal ist.

Daher bestand die Aufgabe, ein magnetisches Tintenkonzentrat der oben genannten gattungsmäßigen Art zu finden, dessen als Dispergiermittel wirkender Polyelektrolyt bezüglich biologischer Abbaubarkeit deutlich verbessert ist. Außerdem war die Aufgabe zu lösen, die Sedimentationsstabilität und damit die Ausbeute des Tintenkonzentrats gegenüber den aus dem Stand der Technik bekannten Polyelektrolyten zu erhöhen.

Erfindungsgemäß wurde die Aufgabe gelöst mit einem magnetischen Tintenkonzentrat mit den im kennzeichnenden Teil des Anspruchs 1 genannten Merkmalen. Weitere Einzelheiten der Erfindung gehen aus den Unteransprüchen sowie der Beschreibung hervor.

Unter dem Begriff biologisch abbaubar wird verstanden, daß aus dem Polyelektrolyten unter dem Einfluß von hydrolytischen, enzymatischen oder photolytischen Bedingungen in Anwesenheit oder Abwesenheit von Sauerstoff die Bildung von Polymerfragmenten erfolgen kann. Der Abbau kann beispielsweise in Kläranlagen, Kompostieranlagen oder unter dem Einfluß lebender Organismen oder in Oberflächengewässern im Erdreich erfolgen.

Erfindungsgemäß besitzen die Polyelektrolyten, deren Molekulargewicht zwischen 500 und 100000 liegt, in der Hauptkette mindestens zwei gleiche oder verschiedene Heteroatome und in der Seitenkette mindestens drei freie Carbonsäure- oder Aminogruppen pro Polymerkette. Die Heteroatome können N, O, P, S, sein und die Carbonsäuregruppen können neutralisiert in Form der NH₄-, Tetramethylammonium-, Li-, Na-, K-, H-, Ca-, Mg-Salze vorliegen. Es hat sich gezeigt, daß die erfindungsgemäßen Polyelektrolyte in Wasser oder in Alkohol mit einer Dielektrizitätskonstante von ≧ 20 oder in Mischungen beider gut dispergierbar sind. Beispiele solcher Polyelektrolyte sind aus den nachfolgenden Versuchsbeispielen zu entnehmen. Die erfindungsgemäßen Polyelektrolyte sind zu über 40 %, vorzugsweise zu über 60 % biologisch abbaubar.

So können Polyasparaginsäure enthaltende Kondensate nach allen bekannten Verfahren beispielsweise beschrieben in der EPB 0 256 366 oder nicht vorveröffentlichten DE-Anmeldung P 43 00 020.7 aus Maleinsäurenahydrid und Ammoniak oder Ammoniumsalzen hergestellt werden, weiterhin aus Asparaginsäure gemäß DE-A 42 17 847 durch Polykondensation mit 0,1 - 10 mol Phosphonsäure pro mol Asparaginsäure oder durch Tempern von Asparaginsäurekristallen bei über 200 °C, bevorzugt 220 - 240 °C, in Form einer Feststoffpolykondensation.

Polyestercarboxylate auf Basis mehrwertiger Carbonsäuren können hergestellt werden, wie in den Patenten DE-B 16 17 122, DE-B 21 47 778, EP-A 0 433 010 sowie den Anmeldungen DE-A 42 13 282, DE-A 41 08 626 und DE-A 40 34 334 beschrieben ist.

Polyacetale können beispielsweise nach einem Verfahren, wie es in dem Patent EP 0 001 004 beschrieben ist, hergestellt werden.

Als Trägermedium für die erfindungsgemäßen Tintenkonzentrate werden, wie bereits oben angeführt, Alkohole mit einer DK von ≧ 20 eingesetzt. Beispielhaft genannt seien Ethylenglykol, Diethylenglykole oder Glycerin, wobei auch Gemische dieser Alkohole mit Wasser eingeschlossen sind.

Die in dem erfindungsgemäßen Tintenkonzentrat eingearbeiteten magnetischen Teilchen können superparamagnetische Feststoffteilchen sein, welche eine spezifische Oberfläche nach BET von 60 bis 130 m²/g, vorzugsweise von 80 bis 110 m²/g aufweisen. Insbesondere sind es superparagmagnetische Feststoffteilchen, die der allgemeinen Formel MᵥMn_{w}ZnₓFe_{y}O_{z} entsprechen, in welche für die Variablen folgende Bedingungen gelten:
M = Co und/oder Ni,
v, w = 0 - 0,998,
x = 0,001 - 0,998,
y = 2,001 - 2,998,
z = 3,001 - 4,
v + w + x = 0,002 - 0,999,
v + w + x + y = 3,
v ≠ 0, falls w = 0; w ≠ 0, falls v = 0.

Derartige superparamagnetische Feststoffteilchen sind beispielsweise in der US 4 810 401 beschrieben.

Ebenso einsetzbar sind aber auch hartmagnetische Feststoffteilchen, beispielsweise Bariumferrite oder andere, wie sie in der parallelen deutschen Anmeldung P 43 27 225 der gleichen Anmelderin beschrieben sind. Derartige hartmagnetische Teilchen haben eine spezifische Oberfläche von 70 bis 200 m²/g, vorzugsweise von 90 bis 120 m²/g und eine Koerzitivkraft zwischen 10 und 500 kA/m, vorzugsweise zwischen 15 und 350 kA/m; die Remanenz derartiger Pigmente oder der Mischung bestehender Pigmente soll über 10 nTm³/g betragen.

Diese erfindungsgemäßen Tintenkonzentrate lassen sich in einfacher Weise herstellen. Hierzu wird eine Mischung aus Wasser oder Alkohol und dem Polyelektrolyten und/oder seinem Alkalisalz in Form einer 20 bis 80 Gew.%igen Lösung mit dem üblicherweise noch feuchten Filterkuchen der magnetischen Feststoffteilchen verrührt und die Suspension anschließend unter der Wirkung hoher Scherkräfte eine halbe bis zwei Stunden dispergiert. Hierbei kann die Temperatur bis zu 70 °C ansteigen. Die Reihenfolge der Zugabe der Komponenten ist beliebig und wirkt sich nicht auf die Eigenschaften des resultierenden Tintenkonzentrats aus. Anschließend wird 5 Minuten bis zwei Stunden bei 200 bis 2000 g zentrifugiert und der kleine Anteil sedimentierter Teilchen abgetrennt.

Das resultierende Produkt entspricht in Aufbau und Eigenschaften dem erfindungsgemäßen magnetischen Tintenkonzentrat, wie aus den nachfolgenden Beispielen hervorgeht, welche die gegenüber aus Vergleichsbeispielen verbesserte Ausbeute zeigt. Die biologische Abbaubarkeit wird beispielsweise anhand der Abnahme der Sauerstoffkonzentration im geschlossenen Flaschentest oder anhand der Abnahme als gelösten organischen Kohlenstoffs im Zahn-Wellens-Test gemäß OECD Guidelines for Testing of Chemicals , Paris 1981, 302 B, bestimmt. Auch der SCAS Test gemäß OECD Guidelines for Testing of Chemicals, Paris 1981, 302 A, ist für die Beurteilung geeignet.

### Beispiele sowie Vergleichsbeispiele

Als magnetische Feststoffteilchen wurden zwei verschiedene Ferrite (Ferrit 1 und Ferrit 2) so wie in der US-Patentschrift 4 810 401 beschrieben durch Fällung einer Eisen/Mangan/Zinksalzlösung mit Natronlauge hergestellt, welche beide die Zusammensetzung Mn_{0,3}Zn_{0,2}Fe_{2,5}O₄ besaßen. Die Ferrite hatten folgende magnetische Eigenschaften

| | BET (m²/g) | M_{m/ρ} (nTm³/g) |
|---|---|---|
| Ferrit 1 | 103 | 82 |
| Ferrit 2 | 103 | 80 |

Die Tintenkonzentrate wurden wie oben beschrieben hergestellt und die Ausbeute wurde nach 5 Minuten Zentrifugieren bei 2000 g Beschleunigung beziehungsweise nach 30 Minuten Zentrifugieren bei 1000 g Beschleunigung (Vergleichsbeispiel 1) bestimmt. Die biologisch abbaubaren Polymerisate werden mittels Natronlauge auf pH 7 neutralisiert. Der K-Wert wurde in 1%iger Lösung in Wasser bei pH 7 gemäß Fikentscher, Cellulosechemie, Band 13 (1932) bei 25 °C bestimmt.

**Tabelle**

| **Vers.-Nr.** | **magnet. Pigment** | **Polyelektrolyt** | **Ausbeute** |
|---|---|---|---|
| Beispiel 1 | Ferrit 1 | Polyasparaginsäure, K-Wert: 25 MG: 7000 | 79 % |
| Beispiel 2 | Ferrit 2 | Polykondensat Citronensäure/EMSlZE E9 (Hydroxypropyl-Kartoffelstärkeether; Hersteller: Emsland-Stärke GmbH) im Molverhältnis 5:1, Na-Salz K-Wert: 24 | 85 % |
| Beispiel 3 | Ferrit 2 | Polykondensat Citronensäure/EMSlZE E9 (Hydroxypropyl-Kartoffelstärkeether; Hersteller: Emsland-Stärke GmbH) im Molverhältnis 5:1, Na-Salz K-Wert: 15 | 91 % |
| Beispiel 4 | Ferrit 2 | Polykondensat Citronensäure/Weinsäure/Na-Salz Molverhältnis 1:1 | 86 % |
| Beispiel 5 | Ferrit 2 | Polyglyoxylsäure-Na-Salz K-Wert: 12; MG: 2500 | 82 % |
| Vergleichsbelspiel 1 | Ferrit 2 | Polyacrylsäure-Na-Salz (Handelsname Sokalan CP 10) MG 4000 | 77 % |
| Vergleichsbeispiel 2 | Ferrit 1 | Polyacrylsäure-Na-Salz (Handelsname Sokalan CP 10) MG 4000 | 77 % |

### Beispiel 6

### Magnetische Flüssigkeit mit biologisch abbaubaren Polyelektrolyten in einem Alkohol.

Als superparamagnetische Feststoffteilchen wurde durch Fällungsreaktion hergestelltes MnZnFerrit der Zusammensetzung Mn_{0,3}Zn_{0,2}Fe_{2,5}O₄ mit folgenden Eigenschaften verwendet: BET-Oberfläche: 96 m²/g, Mₘ/ρ = 87 nTm³/g. Die Fällungsreaktion ist eine dem Fachmann bekannte Reaktion von wäßrigen Eisen-, Mangan- und Zinksalzen mit Lauge. Das aus der Fällungsreaktion gewonnene Pigment wurde filtriert, gewaschen und als feuchter Filterkuchen (MnZnFerritgehalt 31 Gew.%) eingesetzt. 804 g Filterkuchen wurden mit einer Lösung von 50 g Polyasparaginsäure mit einem K-Wert von 29 und 150 g Wasser sowie mit 625 g Ethylenglykol vermengt. Es wurde durch NaOH Zugabe auf pH 9,5 eingestellt und 15 Minuten lang mit einem Ultra Turrax Dispergieraggregat (G45FF) homogenisiert. Anschließend wurde die Mischung 8 Stunden lang in einer Kugelmühle (Dyno Mill) gemahlen. Die gemahlene Magnetflüssigkeit wurde 5 Minuten bei 2000 g in einer Zentrifuge behandelt, wobei nur sehr wenig Sediment abgetrennt wurde. Die Ausbeute an Magnetflüssigkeit betrug 96,8 %. Anschließend wurde das Wasser an einem Rotationsverdampfer bei 80 °C am Vakuum abgezogen. Die derart getrocknete Flüssigkeit hatte eine Sättigungsmagnetisierung von 360 Gauß. Nach 2wöchiger Lagerung konnte am Boden einer 10 cm hohen Schicht der alkoholischen Magnetflüssigkeit keine Sedimentation (< 0,5 %) von Magnetpigment beobachtet werden. Die Flüssigkeit eignet sich zum Beispiel für den Einsatz in Schreibgeräten oder als Trägermedium in Geräten zum Polieren und Läppen von Werkstoffen.

### Beispiel 7

### Magnetische Flüssigkeit mit biologisch abbaubaren Polyelektrolyten in einem Alkohol.

Als superparamagnetische Feststoffteilchen wurde durch Fällungsreaktion hergestelltes Magnetit mit folgenden Eigenschaften verwendet: BET-Oberfläche: 80 m²/g, Mₘ/ρ = 72 nTm³/g. Die Fällungsreaktion ist eine dem Fachmann bekannte Reaktion von wäßrigen Eisen(II, III)salzen mit Lauge. Das aus der Fällungsreaktion gewonnene Pigment wurde filtriert, gewaschen und als feuchter Filterkuchen (Magnetitgehalt 27 Gew.%) eingesetzt. 784 g Filterkuchen wurden mit einer Lösung von 40 g Polyasparaginsäure mit einem K-Wert von 29 und 120 g Wasser sowie mit 500 g Ethylenglykol vermengt. Es wurde durch NaOH Zugabe auf pH 9,2 eingestellt und 10 Minuten lang mit einem Ultra Turrax Dispergieraggregat (G45FF) homogenisiert. Anschließend wurde die Mischung 8 Stunden lang in einer Kugelmühle (Dyno Mill) gemahlen. Die gemahlene Magnetflüssigkeit wurde 5 Minuten bei 2000 g in einer Zentrifuge behandelt, wobei nur sehr wenig Sediment abgetrennt wurde. Die Ausbeute an Magnetflüssigkeit betrug 98,3 %. Anschließend wurde das Wasser an einem Rotationsverdampfer bei 80 °C am Vakuum abgezogen. Die derart getrocknete Flüssigkeit hatte eine Sättigungsmagnetisierung von 350 Gauß. Nach 2wöchiger Lagerung konnte am Boden einer 10 cm hohen Schicht der alkoholischen Magnetflüssigkeit keine Sedimentation (< 0,5 %) von Magnetpigment beobachtet werden. Die Flüssigkeit eignet sich zum Beispiel für den Einsatz in Schreibgeräten oder als Trägermedium in Geräten zum Polieren und Läppen von Werkstoffen.

Derartige Tintenkonzentrate sind anwendbar für Tintenstrahldrucker, Schreibgeräte und Informationsspeicherung. Sie kommen aber auch wegen der biologischen Abbaubarkeit für kosmetische Zwecke in Betracht, falls statt des MnZn-Ferrits einfacher Magnetit genommen wird (schwarze Tinte), ferner für medizinische Anwendungen (MRI-Kontrastmittel) sowie im Bereich der Technik, beispielsweise für das Polieren mit einer Mischung aus magnetischer Flüssigkeit und Schleifmitteln unter Magnetfeldbehandlung.

## Patentansprüche

1. Magnetisches Tintenkonzentrat, im wesentlichen bestehend aus einer Dispersion magnetischer Feststoffteilchen in Wasser oder Alkohol in Gegenwart dispergierender Substanzen, wobei diese aus mindestens einem Polyelektrolyten bestehen, dadurch gekennzeichnet, daß der Polyelektrolyt mit einem Molekulargewicht zwischen 500 und 100000 in der Hauptkette mindestens zwei gleiche oder verschiedene Heteroatome und in der Seitenkette mindestens drei freie Carbonsäuregruppen oder drei freie Aminogruppen pro Polymerkette enthält.

2. Magnetisches Tintenkonzentrat nach Anspruch 1, dadurch gekennzeichnet, daß das Heteroatom N, O, P, S, ist und daß die freien Carbonsäuregruppen neutralisiert in Form der NH₄-, Tetramethylammonium-, Li-, Na-, K-, H-, Ca-, Mg-Salze vorliegen.

3. Magnetisches Tintenkonzentrat nach Anspruch 1, dadurch gekennzeichnet, daß der Alkohol eine Dielektrizitätskonstante von ≧ 20 hat.

4. Magnetisches Tintenkonzentrat nach einem der Ansprüche 1 bis 3, dadurch gekennzeichnet, daß der Polyelektrolyt unter dem Einfluß von hydrolytischen, enzymatischen und/oder photolytischen Bedingungen in Anwesenheit oder Abwesenheit von Sauerstoff unter Bildung von Polymerfragmenten spaltbar ist.
